# EUROPEAN PATENT SPECIFICATION

(11) **EP 2 602 251 B1**
(45) Date of publication and mention of the grant of the patent: **13.01.2016**
(21) Application number: 11814739.6
(22) Date of filing: 05.08.2011
(51) Int. Cl.: C07D 301/12, C07D 303/04, C07D 303/27, C07D 303/28

(54) **METHOD FOR PRODUCING EPOXY COMPOUND BY OXIDATION**
VERFAHREN ZUR HERSTELLUNG EINER EPOXIDVERBINDUNG DURCH OXIDIERUNG
PROCÉDÉ POUR LA PRODUCTION D'UN COMPOSÉ ÉPOXY PAR OXYDATION

(30) Priority: 05.08.2010 JP 2010176377
(43) Date of publication of application: 12.06.2013
(73) Proprietor: Nagase ChemteX Corporation, Osaka-shi Osaka 550-8668 (JP)
(72) Inventor: HASHIZUME Yuki, Tatsuno-shi Hyogo 679-4124 (JP)
(74) Representative: Hoffmann Eitle
(86) International application number: PCT/JP2011/067950
(87) International publication number: WO 2012/018114

(56) References cited:
- JP-A- 9 183 773
- JP-A- 10 212 281
- JP-A- 2003 146 977
- JP-A- 2004 285 055
- JP-A- 2006 525 283
- JP-A- 2007 230 908
- JP-A- 2009 035 541
- JP-A- 2010 159 245
- US-A- 4 012 423
- PANDEY, R.K. ET AL.: 'Eco-friendly synthesis of epichlorohydrin catalyzed by titanium silicate (TS-1) molecular sieve and hydrogen peroxide' CATAL COMMUN vol. 8, no. 3, 2007, pages 379 - 382
- FAN, W. ET AL.: 'Unique solvent effect of microporous crystalline titanosilicates in the oxidation of 1-hexene and cyclohexene' J CATAL vol. 256, no. 1, 2008, pages 62 - 73
- WU, P. ET AL.: 'A novel titanosilicate with MWW structure Catalytic properties in selective epoxidation of diallyl ether with hydrogen peroxide' J CATAL vol. 228, no. 1, 2004, pages 183 - 191
- BONON, A.J. ET AL.: 'Oxidation of alkanes and olefins with hydrogen peroxide in acetonitrile solution catalyzed by a mesoporous titanium- silicate Ti-MMM-2' APPL CATAL A GEN vol. 365, no. 1, 2009, pages 96 - 104

## Description

### TECHNICAL FIELD

The present invention relates to a method for producing an epoxy compound. In particular, the present invention relates to a method for producing an epoxy compound not by the epichlorohydrin process but by oxidation using hydrogen peroxide.

### BACKGROUND ART

With respect to the method for producing an epoxy compound, the epichlorohydrin process is known as a very common method. This process, however, causes contamination of the product with chloro-organic compounds such as chlorohydrin and thus the product has a high total chlorine content. This limits its use for applications such as semiconductors and also requires a complicated purification step to be repeated in order to allow use in such applications.

Production methods by oxidation are known as methods for producing an epoxy compound without using the epichlorohydrin process. Examples thereof include various methods such as oxidation with an organic peracid; oxidation with hydrogen peroxide in the presence of a catalyst such as tungstic acid; oxidation with hydrogen peroxide in the presence of a catalyst such as zeolite; and oxidation with hydrogen peroxide in the presence of a nitrile solvent under a basic condition. Especially, the oxidation with hydrogen peroxide in the presence of a nitrile solvent under a basic condition is advantageous in that this method allows for a relatively safe reaction, wide use, and epoxidation of various raw materials, and the method is also advantageous in cost because no special reagent is used. Known as such a method is, for example, a method of stereoselectively producing 3-carane epoxide, which is an intermediate of insect repellents, by reacting hydrogen peroxide with 3-carene in the presence of acetonitrile and methanol (Patent Literature 1). In this method, it is presumed that the epoxidation reaction proceeds as follows: Acetonitrile is oxidized by hydrogen peroxide under a basic condition due to an alkali metal salt such as sodium carbonate, and then serves as an organic peracid.

In this method, however, hydrogen peroxide is likely to decompose and the oxygen concentration in the system may exceed 20%. Thus, this method is very dangerous in the system including an ignitable solvent, for example. Further, this method has other problems that the reaction temperature is high and that an operation to decompose residual hydrogen peroxide in the system and peroxides generated in the system is required after the reaction, and in this case, the decomposition heat may be high and dangerous by-products may be generated. Further, glass reaction vessels cannot then be used because the reaction is performed under a basic condition with a pH of 9 to 11. In addition, in the case of performing the method in a stainless-steel (SUS) reaction vessel, the surface metal of the reaction vessel serves as a catalyst and disadvantageously accelerates the decomposition of hydrogen peroxide.

Another method is also known in which an epoxy compound is produced from an ethylenic compound with hydrogen peroxide in a stainless-steel (SUS) reaction vessel (Patent Literature 2). This method is a method for producing an epoxy compound by oxidation with hydrogen peroxide in the presence of a catalyst such as tungstic acid.

In this method, however, the reaction in a SUS reaction vessel is made possible by adjusting the ratio between the surface area in the reaction system which is in contact with the gaseous phase in the reaction system and the amount of the reaction liquid in the reaction vessel to a predetermined range, or by passivating the surface in the reaction system which is in contact with the gaseous phase in the reaction system. Thus, this epoxidation reaction in a SUS reaction vessel involves great limitations on the reaction conditions and the like. This method also has another problem that the obtained epoxy compound is hydrolyzed and therefore the yield of the epoxy compound is reduced because the epoxidation is performed under an acidic condition.

### CITATION LIST

### PATENT LITERATURE

Patent Literature 1: JP H10-212281 A
Patent Literature 2: JP H09-183773 A

Patent Literature 3: JP 2003-146977 A discloses a method for producing an epoxy compound that comprises oxidizing the compound having at least one ethylenic double bond with the oxidizing agent. The method is further characterized by using a heteropolyoxometalate anion salt which has two defective structure sites and contains a silicon atom as a heterogeneous atom, as a homogeneous catalyst.

Patent Literature 4: JP 2004-285055 A discloses a method for producing propylene oxide by reacting propylene and hydrogen peroxide in a nitrile solvent using the crystalline titanosilicate having a ring of 12 or more members of oxygen and pore structure as a catalyst, wherein the mixture of water/nitrile compound having a weight ratio of 0/100 to 20/80 is fed to a reactor.

Patent Literature 5: JP 2006-525283 A discloses a process for preparing propylene oxide, at least comprising the steps (i) and (ii): (i) providing a catalyst comprising at least one porous oxidic material, (ii) reaction of propene with a hydroperoxide in at least one nitrile as solvent or in an at least one nitrile-containing solvent mixture, in the presence of the catalyst according to (i), characterized in that the at least one porous oxidic material is a zeolite X-ray-assignment to the MWW type.

Patent Literature 6: JP 2007-230908 A discloses a method for producing an epoxy compound which comprises mixing an olefin compound with hydrogen peroxide, passing the resultant mixture in a microreactor packed with an oxidation catalyst to epoxidize the olefin compound. Preferably, the mixing of the olefin compound with the hydrogen peroxide is carried out by using a micromixer, and at least one kind selected from the group consisting of a zeolite catalyst, a tungsten polyoxomethalate carried by a carrier, a metal compound carried by a carrier, a metal oxide and a metal oxide carried by a carrier is preferably used as the oxidation catalyst.

Patent Literature 7: JP 2009-035541 A discloses a method for producing the propylene oxide which comprises a process of reacting hydrogen peroxide with propylene in the presence of a titanosilicate catalyst in acetonitrile or a mixture of acetonitrile with water to obtain a reaction mixture containing the propylene oxide, a process of gas liquid separation of the reaction mixture obtained by the above reaction process, to the gas and reaction liquid, and a process of distillation for separating the reaction liquid prepared by the process of the gas liquid separation into a column top liquid containing the propylene oxide and a column bottom liquid containing the acetonitrile or the acetonitrile and water by distillation.

Patent Literature 8: JP 2010-159245 A discloses a method for producing the oxidized compound which comprises a process of oxidizing an organic compound by reacting the organic compound with an oxidizing agent in the presence of a titanosilicate (I) obtained by making contact of a titanosilicate (II) having the following X-ray diffraction pattern with a structure-regulating agent, or its silylated compound. The X-ray diffraction pattern (spacing of lattice plane, d/ not A) 12.4+-0.8, 10.8+-0.3, 9.0+-0.3, 6.0+-0.3, 3.9+-0.1, 3.4+-0.1.

Patent Literature 9: US 4012423 A discloses a vapor phase process for the synthesis of oxiranes from vicinal hydroxyester in the presence of a basic material.

Non Patent Literature 1: Catal Commun, 2007, vol. 8, no. 3, pages 379 to 382, discloses that in a mixed solvent system of acetonitrile and methanol, titanium silicate molecular sieve, TS-1, having MFI topology, catalyses the epoxidation of allyl chloride to the corresponding epichlorohydrin using dilute hydrogen peroxide as an oxidizing agent.

Non Patent Literature 2: J Catal, 2008, vol. 256, no. 1, pages 62 to 73, discloses the influence of solvent on the activity, epoxide selectivity and H₂O₂ efficiency of three types of zeolites - TS-1, Ti-Beta and Ti-MWW - in the liquid-phase epoxidation of 1-hexene and cyclohexene.

Non Patent Literature 3: J Catal, 2004, vol. 228, no. 1, pages 183 to 191, discloses the catalytic activity and selectivtity of Ti-MWW in the epoxidation of diallyl ether with hydrogen peroxide to allyl glycidyl ether and diglycidyl ether by a comparison with those of TS-1 and the issues concerning the consecutive reaction and the selective production of allyl glycidyl ether.

Non Patent Literature 4: Appl Catal A Gen, 2009, vol. 365, no. 1, pages 96 to 104, discloses a mesoporous titanium-silicate Ti-MMM-2 that catalyses oxidation of alkanes and olefins by H₂O₂ in acetonitrile solution at 60 °C. The oxidation occurs *via* the formation of a 'Ti-OOH' species on the catalyst surface which either epoxidises a nucleophilic double bond or generates, after O-O bond splitting, hydroxyl radical.

### TECHNICAL PROBLEM

The present invention aims to provide a method for producing an epoxy compound by oxidation using hydrogen peroxide, in which the generation of oxygen due to the decomposition of hydrogen peroxide can be suppressed in the system, and which does not require complicated post-treatment, is excellent in reactivity, and allows the reaction to be performed in a SUS vessel.

### SOLUTION TO PROBLEM

The present inventor has performed intensive studies for solving the above problems. Thus, the inventor has found that when an epoxidation reaction of an olefin compound and hydrogen peroxide in the presence of a nitrile compound is performed in the presence of a silicate as a basic substance, the generation of oxygen due to the decomposition of hydrogen peroxide can be suppressed in the system, and the reaction rate is accelerated; moreover, the reaction does not require complicated post-treatment and can be performed in a SUS vessel, thereby completing the present invention.

The present invention relates to a method for producing an epoxy compound, the method comprising reacting an olefin compound with hydrogen peroxide in the presence of a nitrile compound and a silicate,
wherein the silicate is at least one selected from sodium silicate and potassium silicate.

The nitrile compound is preferably at least one selected from acetonitrile, propionitrile and benzonitrile

The reaction is preferably performed in the presence of an alcohol solvent.

### ADVANTAGEOUS EFFECTS OF INVENTION

The method for producing an epoxy compound according to the present invention allows to keep the concentration of hydrogen peroxide in the system low to suppress the generation of oxygen due to the decomposition of hydrogen peroxide, and therefore the epoxidation reaction can be safely performed. Further, this method is suitable for industrialization because the method requires a small amount of hydrogen peroxide, allows for a short reaction time and a low reaction temperature, does not require complicated post-treatment, and allows the epoxidation reaction to be performed in a SUS vessel. Furthermore, the method requires no chloro-organic compound as a reagent, and therefore the total chlorine content can be limited to the amount originally contained in the material compounds, that is, about few hundred ppm.

In addition, the resulting epoxy compound has a very low total chlorine content of about few hundred ppm, and thus can be suitably used for electronic materials.

### DESCRIPTION OF EMBODIMENTS

The following will describe the present invention in detail.

The method for producing an epoxy compound according to the present invention is characterized by comprising reacting an olefin compound with hydrogen peroxide in the presence of a nitrile compound and a silicate, wherein the silicate is at least one selected from sodium silicate and potassium silicate.

First of all, the components used in the present invention will be described one by one.

The olefin compound used in the present invention may be any one having a carbon-carbon double bond in its molecule, and may also be an olefin compound having multiple carbon-carbon double bonds. The olefin compound may be a low-molecular-weight compound or may be a high-molecular-weight compound, and may have either a cis or trans structure, or both of them may be mixed. The olefin compound may further have any of functional groups and cyclic structures such as unsaturated bonds, a hydroxy group, an alkoxyl group, a carbonyl group, a carboxyl group, an amido group, an ester group, and halogen atoms in its molecular chain or at its molecular terminal. Examples thereof include 1-butene, 1-pentene, cyclohexene, 1,7-octadiene, 1,4-cyclohexane dimethanol diallyl ether, trimethylolpropane triallyl ether, pentaerythritol tetraallyl ether, ditrimethylolpropane tetraallyl ether, and dipentaerythritol hexaallyl ether.

The molecular weight of the olefin compound (the number average molecular weight (Mn) in the case of a high-molecular-weight compound) is not particularly limited, but it is preferably 100 to 3000, in general.

In the present invention, hydrogen peroxide is used in the form of an aqueous solution. The concentration of hydrogen peroxide in the aqueous solution is not particularly limited. Its upper limit is preferably 60%, and more preferably 35%, whereas the lower limit of the concentration is preferably 5%, and more preferably 10%. A concentration of lower than 5% tends to cause reduction in the reaction rate and in the batch efficiency. Conversely, a concentration of higher than 60% tends to cause difficulty in controlling the internal temperature upon reaction and to easily cause abnormal decomposition of hydrogen peroxide, leading to an increase in risk.

The amount of hydrogen peroxide is not particularly limited, and depends on factors such as the types of olefin compound and silicate, and reaction conditions. The amount in terms of hydrogen peroxide is preferably 1 to 10 equivalents per equivalent of the carbon-carbon double bond in the olefin compound. The upper limit of the amount is preferably 5 equivalents, and more preferably 2 equivalents, whereas the lower limit of the amount is preferably 1 equivalent, and more preferably 1.5 equivalents. If hydrogen peroxide is used in an amount less than 1 equivalent, the reaction tends to insufficiently proceed. If hydrogen peroxide is used in an amount more than 10 equivalents, control of the reaction and assurance of the safety tend to be difficult.

The nitrile compound used in the present invention is not particularly limited, and may be any of aliphatic and aromatic nitrile compounds. Each of these may be used alone, or two or more of these may be used in combination. Examples of the aliphatic nitrile compounds include acetonitrile, propionitrile, n-butyronitrile, isobutyronitrile, malononitrile, adiponitrile, monochloroacetonitrile, dichloroacetonitrile, and trichloroacetonitrile. Examples of the aromatic nitrile compounds include benzonitrile, tolunitrile, chlorobenzonitrile, and fluorobenzonitrile. Preferred among these are acetonitrile, propionitrile and benzonitrile, from the viewpoints of their good reactivity and easy removability of the nitrile-derived by-products, and particularly preferred is acetonitrile.

The amount of the nitrile compound is not particularly limited, and depends on factors such as the types of olefin compound and silicate, and reaction conditions. The amount is preferably 1 to 10 equivalents per equivalent of the carbon-carbon double bond in the olefin compound. The upper limit of the amount is preferably 10 equivalents, and more preferably 3 equivalents, whereas the lower limit of the amount is preferably 1 equivalent, and more preferably 1.5 equivalents. If the nitrile compound is used in an amount less than 1 equivalent, the reaction tends to insufficiently proceed. If the nitrile compound is used in an amount more than 10 equivalents, the reactivity tends to be reduced.

The silicate used in the present invention is at least one selected from sodium silicate and potassium silicate. Each of the silicates may be used alone, or in combination. Sodium silicate and potassium silicate, are silicates of alkali metals, which are used from the viewpoints of their high reactivity and easy availability. The silicate may be a commercially available product, and may be in the form of a solid or may be in the form of an aqueous solution. When the silicate is used, the conditions inside the reaction system are made weakly basic and thus the epoxy group in the epoxy compound produced is less likely to hydrolyze. Consequently, a high yield can be achieved.

The amount of the silicate depends on factors such as reaction conditions, and is not particularly limited. It is preferably 0.001 to 2 equivalents per equivalent of the carbon-carbon double bond in the olefin compound. The upper limit of the amount is preferably 2 equivalents, and more preferably 0.5 equivalents, whereas the lower limit of the amount is preferably 0.001 equivalents, and more preferably 0.01 equivalents. If the silicate is used in an amount less than 0.001 equivalents, the reactivity may be reduced. If the silicate is used in an amount more than 2 equivalents, the epoxy may easily hydrolyze, and the product may be difficult to isolate and purify after the reaction, likely resulting in reduction in yield.

The following will describe the reaction conditions.

In the present invention, a reaction solvent may be used. The reaction solvent is not particularly limited. Examples thereof include alcohol solvents such as methanol, ethanol, propanol and isopropanol; ketones such as acetone and methyl ethyl ketone; amides such as N,N-dimethylformamide and N,N-dimethylacetamide; and 1,2-dimethoxyethane and diethylene glycol dimethyl ether. Each of these solvents may be used alone, or two or more of these may be used in combination. Preferred among these are alcohol solvents because of their miscibility with water and availability at low cost. The amount of the reaction solvent is not particularly limited. It is preferably 10 to 1000 parts by weight for each 100 parts by weight of the olefin compound. The solvent used in an amount less than 10 parts by weight may not allow the raw materials to be mixed well in the system, and therefore tends to lead to reduced reactivity. The solvent used in an amount more than 1000 parts by weight may cause reduction in the concentration of the raw materials in the system, and therefore tends to lead to a reduced reaction rate.

The reaction temperature is not particularly limited. In the present invention, the reaction may be performed at relatively low temperatures. The reaction temperature is preferably 0°C to 100°C, particularly preferably 20°C to 80°C, and further preferably 20°C to 45°C. A temperature higher than 100°C tends to accelerate the decomposition of hydrogen peroxide and the hydrolysis of the epoxy produced. A temperature lower than 0°C tends to cause an insufficient reaction rate, likely resulting in incomplete reaction.

The reaction time depends on factors such as the scale of reaction. It may generally be within the range from 1 to 72 hours, and is preferably 2 to 24 hours.

In the present invention, an olefin compound and hydrogen peroxide are reacted in the presence of a nitrile compound and a silicate. The order of adding these compounds is not particularly limited. In a typical reaction, an olefin compound and a nitrile compound are first mixed in a reaction solvent; then a silicate is added thereto; a hydrogen peroxide solution is dropwise added and the mixture is stirred, with careful attention to the temperature of the mixture; after the reaction, the silicate is optionally filtered off and then common operations such as water washing and concentration are performed, whereby an epoxy compound is obtained.

The whole silicate needs not to be dissolved and it has only to be dispersed in the reaction solution by stirring. Moreover, in the present invention, since hydrogen peroxide is rapidly consumed in the epoxidation reaction, the concentration of hydrogen peroxide in the system does not excessively increase even when the required amount of hydrogen peroxide is added at once. Alternatively, hydrogen peroxide may be added in several portions, or may be added continuously.

In the present invention, the silicate increases the reaction rate of hydrogen peroxide so that hydrogen peroxide is rapidly consumed in the epoxidation reaction, in comparison with the case of using sodium carbonate. Thus, it leads to various advantages such as a shorter reaction time, reduction in the concentration of hydrogen peroxide in the system, suppression of the decomposition of hydrogen peroxide, suppression of the generation of oxygen due to the decomposition of hydrogen peroxide in the system, reduction in the amount of hydrogen peroxide, no need of the step of decomposing residual peroxides after the reaction, and a lower reaction temperature. Therefore, the reaction can be performed more safely and moreover, as the concentration of hydrogen peroxide in the system is low, a SUS vessel can be used as a reaction vessel. Examples of the SUS vessel include stainless-steel vessels such as those made of SUS304, 304L, 316, and 316L.

### EXAMPLES

The following will describe the present invention referring to, but not limited to, examples.

### (Gas chromatography (GC))

Gas chromatography was performed using GC-2010 (Shimadzu Corp.).

### (Completion of reaction)

The point when the degree of reaction (degree of epoxidation), which was measured using GC-2010 (Shimadzu Corp.), reached 95% or higher was defined as the end point of the reaction.

### (Concentration of peroxides)

The concentration of peroxides in the reaction mixture was measured as follows. Titration was performed using a solution of cerium (IV) sulfate with o-phenanthroline as an indicator. The point when the color of the indicator turned from red to blue was defined as the end point of titration (cerium (IV) method).

### (Total chlorine content)

The total chlorine content in the product was determined in accordance with JIS K7243-3.

### (Degree of reaction)

GC measurement was performed using GC-2010 (Shimadzu Corp.) to determine the ratio in area between the material (olefin compound) and the product (epoxy compound), and the degree of reaction was then calculated based on the ratio in area.

### (Example 1)

A 1-L SUS four-necked flask equipped with a reflux condenser, a nitrogen-introducing pipe and a thermometer was charged with pentaerythritol tetraallyl ether (148 g (0.5 mol)), methanol (200 g (6.25 mol)), acetonitrile (164.2 g (4 mol)) and sodium silicate (KISHIDA CHEMICAL Co., Ltd., 61 g (0.5 mol)). Then, aqueous hydrogen peroxide (35%) (388.6 g (4 mol)) was slowly dropwise added over five hours using a dropping funnel under stirring, during which the internal temperature was adjusted to 40°C. Thereafter, the mixture was reacted for four hours under stirring. The concentration of peroxides in the system at the end of the reaction was 0.27%. Next, the reaction mixture was extracted with toluene, then washed with water to remove peroxides, and concentrated to give pentaerythritol tetraglycidyl ether at a yield of 62% and a GC purity of 98.2%. Moreover, the obtained pentaerythritol tetraglycidyl ether had a total chlorine content of 120 ppm.

### (Example 2)

A 1-L SUS four-necked flask equipped with a reflux condenser, a nitrogen-introducing pipe and a thermometer was charged with trimethylolpropane triallyl ether (127.2 g (0.5 mol)), methanol (200 g (6.25 mol)), acetonitrile (123.1 g (3 mol)) and sodium silicate (KISHIDA CHEMICAL Co., Ltd., 45.8 g (0.38 mol)). Then, aqueous hydrogen peroxide (35%) (291.4 g (3 mol)) was slowly dropwise added over five hours using a dropping funnel under stirring, during which the internal temperature was adjusted to 40°C. Thereafter, the mixture was reacted for eight hours under stirring. The concentration of peroxides in the system at the end of the reaction was 0.21%. Next, the reaction mixture was extracted with toluene, then washed with water to remove peroxides, and concentrated to give trimethylolpropane triglycidyl ether at a yield of 80% and a GC purity of 95.4%. Moreover, the obtained trimethylolpropane triglycidyl ether had a total chlorine content of 200 ppm.

### (Example 3)

A 1-L SUS four-necked flask equipped with a reflux condenser, a nitrogen-introducing pipe and a thermometer was charged with 1,4-cyclohexane dimethanol diallyl ether (112.2 g (0.5 mol)), methanol (176 g (5.5 mol)), acetonitrile (102.6 g (2.5 mol)) and sodium silicate (KISHIDA CHEMICAL Co., Ltd., 61 g (0.5 mol)). Then, aqueous hydrogen peroxide (35%) (194.3 g (2 mol)) was slowly dropwise added over five hours using a dropping funnel under stirring, during which the internal temperature was adjusted to 40°C. Thereafter, the mixture was reacted for six hours under stirring. The concentration of peroxides in the system at the end of the reaction was 0.18%. Next, the reaction mixture was extracted with toluene, then washed with water to remove peroxides, and concentrated to give 1,4-cyclohexane dimethanol diglycidyl ether at a yield of 85.5% and a GC purity of 99.4%. Moreover, the obtained 1,4-cyclohexane dimethanol diglycidyl ether had a total chlorine content of 210 ppm.

### (Example 4)

A 1-L SUS four-necked flask equipped with a reflux condenser, a nitrogen-introducing pipe and a thermometer was charged with 1,7-octadiene (121.2 g (1.1 mol)), methanol (160 g (5 mol)), acetonitrile (185 g (4.5 mol)) and sodium silicate (KISHIDA CHEMICAL Co., Ltd., 61 g (0.5 mol)). Then, aqueous hydrogen peroxide (35%) (408 g (4.2 mol)) was slowly dropwise added over five hours using a dropping funnel under stirring, during which the internal temperature was adjusted to 40°C. Thereafter, the mixture was reacted for eight hours under stirring. The concentration of peroxides in the system at the end of the reaction was 0.09%. Next, the reaction mixture was extracted with toluene, then washed with water to remove peroxides, and concentrated to give 1,7-octadiene diepoxide at a yield of 55% and a GC purity of 97.0%. Moreover, the obtained 1,7-octadiene diepoxide had a total chlorine content of 140 ppm.

### (Example 5)

The experiment was performed in the same manner as in Example 1, except that benzonitrile (417.5 g (4 mol)) was used instead of acetonitrile (164.2 g (4 mol)). The concentration of peroxides in the system at the end of the reaction was 0.26%. As benzonitrile was used instead, crystalline benzamide precipitated as a by-product; however, by isolation and purification, the target product pentaerythritol tetraglycidyl ether was obtained at a yield of 50.2% and a GC purity of 96.9%. Moreover, the obtained pentaerythritol tetraglycidyl ether had a total chlorine content of 420 ppm.

### (Example 6)

The experiment was performed in the same manner as in Example 1, except that a 27% aqueous solution of potassium silicate (KISHIDA CHEMICAL Co., Ltd., 285.7 g (0.5 mol)) was used instead of sodium silicate (KISHIDA CHEMICAL Co., Ltd., 61 g (0.5 mol)). The concentration of peroxides in the system at the end of the reaction was 0.12%. Next, the reaction mixture was extracted with toluene, then washed with water to remove peroxides, and concentrated to give pentaerythritol tetraglycidyl ether at a yield of 59.8% and a GC purity of 98.5%. Moreover, the obtained pentaerythritol tetraglycidyl ether had a total chlorine content of 190 ppm.

### (Example 7)

The experiment was performed in the same manner as in Example 1, except that the reaction temperature was 25°C. In this case, the reaction was continued for another eight hours to complete the reaction. The concentration of peroxides in the system at the end of the reaction was 0.17%. Next, the reaction mixture was extracted with toluene, then washed with water to remove peroxides, and concentrated to give pentaerythritol tetraglycidyl ether at a yield of 57.4% and a GC purity of 97.9%. Moreover, the obtained pentaerythritol tetraglycidyl ether had a total chlorine content of 130 ppm.

### (Comparative Example 1)

The experiment was performed in the same manner as in Example 1, except that sodium carbonate (KISHIDA CHEMICAL Co., Ltd., 42 g (0.5 mol)) was used instead of sodium silicate and the amount of hydrogen peroxide added was 485.8 g (5 mol). In this case, after the dropwise addition of hydrogen peroxide, the reaction was performed for nine hours to complete the reaction. The concentration of peroxides in the system at the end of the reaction was 2.54%. Next, the reaction mixture was extracted with toluene and the residual peroxides were then decomposed by a 10% aqueous solution (50 g) of sodium sulfite. The resulting product was then washed with water to give pentaerythritol tetraglycidyl ether at a yield of 55.0% and a GC purity of 98.2%. Moreover, the obtained pentaerythritol tetraglycidyl ether had a total chlorine content of 210 ppm.

### (Comparative Example 2)

The experiment was performed in the same manner as in Example 7, except that sodium carbonate (KISHIDA CHEMICAL Co., Ltd., 42 g (0.5 mol)) was used instead of sodium silicate. In this case, after 14 hours of reaction following the dropwise addition of hydrogen peroxide, the reaction would not proceed any further. The degree of reaction at the termination of the reaction was 45.6%.

### INDUSTRIAL APPLICABILITY

According to the present invention, it is possible to keep the concentration of hydrogen peroxide in the system low to suppress the generation of oxygen due to the decomposition of hydrogen peroxide, and therefore the epoxidation reaction can be safely performed. Further, the epoxidation reaction can be performed in a SUS vessel and does not require complicated post-treatment. Thus, the present invention is suitable for industrialization. In addition, compared with a method including condensation of epichlorohydrin, the present invention can be suitably used in the field of electronic materials since the reaction system does not contain chlorine.

## Claims

1. A method for producing an epoxy compound, the method comprising
reacting an olefin compound with hydrogen peroxide in the presence of a nitrile compound and a silicate,
wherein the silicate is at least one selected from sodium silicate and potassium silicate.

2. The method for producing an epoxy compound according to claim 1,
wherein the nitrile compound is at least one selected from acetonitrile, propionitrile and benzonitrile.

3. The method for producing an epoxy compound according to any one of claims 1 or 2,
wherein the reaction is performed in the presence of an alcohol solvent.

## Patentansprüche

1. Verfahren zur Herstellung einer Epoxyverbindung, wobei das Verfahren umfasst
Umsetzen einer Olefinverbindung mit Wasserstoffperoxid in der Gegenwart einer Nitrilverbindung und eines Silikats,
worin das Silikat mindestens eines ist, das ausgewählt ist aus Natriumsilikat und Kaliumsilikat.

2. Verfahren zur Herstellung einer Epoxyverbindung gemäß Anspruch 1, worin die Nitrilverbindung mindestens eine ist, die ausgewählt ist aus Acetonitril, Proprionitril und Benzonnitril.

3. Verfahren zur Herstellung einer Epoxyverbindung gemäß irgendeinem der Ansprüche 1 oder 2,
worin die Umsetzung in der Gegenwart eines Alkohol-Lösungsmittel durchgeführt wird.

## Revendications

1. Procédé pour fabriquer un composé époxy, le procédé comprenant
la réaction d'un composé oléfine avec du peroxyde d'hydrogène en présence d'un composé nitrile et d'un silicate,
dans laquelle le silicate est au moins l'un choisi parmi le silicate de sodium et le silicate de potassium.

2. Le procédé pour fabriquer un composé époxy selon la revendication 1,
dans lequel le composé nitrile est au moins l'un choisi parmi l'acétonitrile, le propionitrile et le benzonitrile.

3. Le procédé pour fabriquer un composé époxy selon l'une quelconque des revendications 1 ou 2,
dans lequel la réaction est effectuée en présence d'un solvant alcoolique.
